(19) Europäisches Patentamt
European Patent Office
Office européen des brevets

(11) EP 4 074 251 A1

(12) EUROPEAN PATENT APPLICATION

(43) Date of publication:
19.10.2022 Bulletin 2022/42

(21) Application number: 21167783.6

(22) Date of filing: 12.04.2021

(51) International Patent Classification (IPC):
A61B 5/026 (2006.01)    A61B 5/0507 (2021.01)
A61B 5/00 (2006.01)     A61B 5/11 (2006.01)

(52) Cooperative Patent Classification (CPC):
A61B 5/0261; A61B 5/0507; A61B 5/1114;
A61B 5/1121; A61B 5/4875; A61B 5/4878;
A61B 5/01; A61B 5/02055; A61B 5/6824;
A61B 5/6828; A61B 5/6833; A61B 5/7267

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• HIWALE, Sujitkumar
Eindhoven (NL)
• BERA, Deep
Eindhoven (NL)

(74) Representative: Philips Intellectual Property &
Standards
High Tech Campus 52
5656 AG Eindhoven (NL)

(54) TISSUE PERFUSION ANALYSIS

(57)    A means is provided for determining perfusion information relating to a body part of a subject based on THz data relating to the body part acquired over the course of an examination period, during which the body part is placed in a number of different positions or poses, and based on positioning or movement information relating to the body part during that examination period.

FIG. 1

**Description**

FIELD OF THE INVENTION

The present invention relates to determination of perfusion information, in particular based on THz sensing data.

BACKGROUND OF THE INVENTION

Perfusion can be defined as an amount of blood exchange in a tissue per unit time. Poor (i.e. low) tissue perfusion can result in ischemia, tissue damage, infarction and ulceration. Excess (high) perfusion, i.e. accumulation of fluid in a tissue, can lead to oedema. For healthy functioning of a biological system, it is very important for perfusion to be maintained at an optimal level in relation to the metabolic demand of the system. This is crucial as both hypo-perfusion (less than required for physiological functioning) or hyper-perfusion (more than is required) can have a deleterious effect on tissue or organ. Where perfusion pathology occurs, optimal perfusion may be restored and maintained at a tissue level by direct or indirect medical or surgical interventions. However, an important prerequisite for such interventions is an ability to detect and quantify perfusion abnormality as early as possible.

In the lower extremities, poor perfusion may result from arterial, venous or pathologies of others systems (e.g., heart failure, renal pathologies). Lower extremities are a common site for peripheral arterial disease (PAD), which is characterized by a progressive narrowing of the arteries. Narrowing of the arteries can lead to reduced tissue perfusion. PAD is a major health challenge responsible for significant age-related patient morbidity. It is estimated that PAD affects 4% of adults aged 40 years or older and 14.5% of adults aged 70 years or older in the United States of America (USA).

The key characterizing symptom of PAD is leg pain during physical activity. In chronic cases it may lead to ulceration and/or necrosis. However, not all cases of PAD have symptomatic presentation. It is estimated that 50% of PAD cases are symptomless.

Another perfusion-related pathology is hypovolemia. This occurs from depletion of intravascular volume. This can also present with a reduced tissue perfusion. Depending upon the amount of fluid loss, hypovolemia can also present with generalized weakness and leg pain. There are also many other disease conditions which present with leg pain. Thus, it is difficult to differentially diagnose PAD or hypovolemia based solely on medical history and symptoms.

Excess fluid collection in tissue is known as oedema. Lower extremities are the most common oedema site. Oedema can be caused by a local pathology or by a generalized pathology. Local causes of oedema may include venous obstruction, as in the case of deep vein thrombosis (DVT) and varicose veins, as well as compartment syndrome or bone fracture. Generalized oedema can be caused by other generalized pathologies such as congestive heart failure, renal failure, and hypo-proteinaemia. Therefore, there can be different causes of lower leg oedema.

In conventional practice, perfusion abnormalities are diagnosed based on clinical history and physical examination. Weak or absent pulse in peripheral arteries provides an indication of reduced tissue perfusion. However, such symptoms manifest late in disease progression, so do not permit early detection of perfusion abnormality. Thus diagnosis is not possible before a significant amount of fluid is already accumulated in the tissue. Furthermore, it is not possible to achieve quantification of perfusion changes based solely on clinical examination. Furthermore, clinical assessment for oedema is subjective in nature, making assessment uncertain.

Physical examination is usually followed by medical imaging and laboratory investigations. Ultrasound is the most common imaging modality for perfusion abnormalities. Ultrasound is inexpensive and nonionizing but requires a skilled operator. CT angiography is relatively inexpensive and has good soft-tissue contrast imaging capabilities, but administers a significant dose of ionizing radiation per scan. MRI angiography is nonionizing, offers excellent soft tissue contrast imaging, but is expensive, has limited portability and is often time consuming.

In the current state of the art, no beside examination method is available, for instance for use of a general physician (GP), to permit quick screening and quantification of tissue perfusion.

It is well known that optical properties of biological tissues are able to provide information relating to their structure. The use of Terahertz spectroscopy has previously been used to assess optical properties of tissue.

Terahertz (THz) spectroscopy has been actively explored to study optical properties of human tissues. THz sensing comprises propagation of THz frequency electromagnetic waves into tissues. It is known by a variety of names including submillimeter radiation, terahertz waves, tremendously high frequency (THF), T-rays, T-waves, T-light, T-lux or THz - . Terahertz radiation consists of electromagnetic waves within the ITU-designated band of frequencies from 0.3 to 3 terahertz (THz), although the upper boundary is somewhat arbitrary and is considered by some sources to be 30 THz. One terahertz is $10^{12}$ Hz or 1000 GHz. Wavelengths of radiation in the terahertz band correspondingly range from 1 mm to 0.01 mm.

Although terahertz radiation penetrates for some distance through body tissue like x-rays, the radiation is non-ionizing.

THz waves are strongly absorbed by hydrogen bonds in water. Therefore cells and tissues with different water content display distinctive responses to THz radiation, making it extremely sensitive to subtle change in water or blood content in tissue. Blood is ~92% of water by volume.

A study has shown that THz spectroscopy is sufficiently precise to recognize a relative change of 0.06% in water concentration. Studies for bio-medical applications have demonstrated that the THz method is sensitive to even 0.5% of change in water volume. Therefore, this technique has previously been proposed for *in vivo* non-invasive monitoring of subtle hydration changes in human tissues.

Terahertz has been used for detection skin, breast, colon, and skin pathologies. Recent studies have successfully demonstrated use of THz for screening diabetic foot syndrome based on changes in tissue hydration. THz imaging has been also used for early, and accurate detection of comeal diseases by means of studying corneal tissue water content. Promising results of THz detection have been obtained in the examination of gastrointestinal tract tumors. Some studies suggest that THz spectroscopy could be a potential screening technique for colorectal, hepatic, and gastric carcinomas.

Reference is made to the paper: N. Bajwa et al., "Terahertz Imaging of Cutaneous Edema: Correlation With Magnetic Resonance Imaging in Burn Wounds," IEEE Trans Biomed Eng, vol. 64, no. 11, pp. 2682-2694, 2017. Bawja et al., have demonstrated successful use of THz spectroscopy for *in vivo* visualization and quantification of total water content (TWC) in a burn-induced model of oedema. TWC is a direct indicator of oedema. The above paper has demonstrated that THz imaging tracks movement of water at dermal tissue depths (greater than ~ 300 $\mu$m) that are relevant to oedema assessment. It has further showed that THz imaging can offer rapid measurements of TWC for potential oedema assessment and provide these measurements earlier than existing clinical and research techniques, such as clinical assessment, MRI, and water displacement studies.

Researchers have proposed different models to derive tissue water content (TWC) using THz. Respective models are outlined for example in each of the following papers:

- G. G. Hernandez-Cardoso et al., "Terahertz imaging for early screening of diabetic foot syndrome: A proof of concept," Sci Rep, vol. 7, p. 42124, 06 2017.
- N. Bajwa et al., "Terahertz Imaging of Cutaneous Edema: Correlation With Magnetic Resonance Imaging in Burn Wounds," IEEE Trans Biomed Eng, vol. 64, no. 11, pp. 2682-2694,2017.
- Q. Sun, Y. He, K. Liu, S. Fan, E. P. J. Parrott, and E. Pickwell-MacPherson, "Recent advances in terahertz technology for biomedical applications," Quant Imaging Med Surg, vol. 7, no. 3, pp. 345-355, Jun. 2017.
- S. I. Alekseev, I. Szabo, and M. C. Ziskin, "Millimeter wave reflectivity used for measurement of skin hydration with different moisturizers," Skin Research and Technology, vol. 14, no. 4, pp. 390-396, 2008.

Details of one such model is summarized below. This model is outlined in the paper G. G. Hernandez-Cardoso et al., "Terahertz imaging for early screening of diabetic foot syndrome: A proof of concept," Sci Rep, vol. 7, p. 42124, 06 2017. In this model, hydrated skin is considered as a combination of dehydrated skin and water. The complex dielectric properties of water are well-known. Experiments were conducted to characterize dielectric properties of dehydrated skin. Based on this, a complex dielectric function of human skin ($\varepsilon_R$ and $\varepsilon_I$) can be approximated by,

$$\varepsilon_R = -0.16\frac{\varepsilon_0}{\text{THz}^2}f^2 + 0.13\frac{\varepsilon_0}{\text{THz}}f + 2.3\varepsilon_0 \qquad (1)$$

$$\varepsilon_I = -0.10\frac{\varepsilon_0}{\text{THz}^2}f^2 + 0.36\frac{\varepsilon_0}{\text{THz}}f + 0.08\varepsilon_0 \qquad (2)$$

where $\varepsilon_R$ and $\varepsilon_I$ are the real and imaginary parts of the complex dielectric function, *f* is the frequency in THz, and 'THz' is the THz sensing signal.

Based on this, the dielectric function of the hydrated skin may be given by:

$$\sqrt[3]{\varepsilon_{\text{mix}}(\omega,\eta)} = \eta\sqrt[3]{\varepsilon_W(\omega)} + (1-\eta)\sqrt[3]{\varepsilon_{DS}(\omega)} \qquad (3)$$

where $\eta$ is the volumetric fraction of water, $\omega$ is the angular frequency of the THz radiation, $\varepsilon_W$ is the well-known dielectric function of water, $\varepsilon_{DS}$ is the dielectric function of the dehydrated skin, and $\varepsilon_{\text{mix}}$ is the dielectric function of the hydrated skin (this representing effectively a mixture of the dielectric functions of dry skin and water).

The model permits obtaining of hydration information for each pixel of a constructed terahertz image. This is achieved using a transfer function which can be calculated from experimental data, or can be modelled theoretically as a function of the water volumetric fraction $\eta$.

Although research in the field has identified means for deriving tissue hydration information from THz sensing data, the link between this data and perfusion abnormalities is missing. In particular, it is not possible to perform differential diagnosis of perfusion abnormalities based solely on tissue hydration information provided by THz.

An improved means for utilizing THz sensing data for assessment of tissue perfusion would therefore be of value.

SUMMARY OF THE INVENTION

The invention is defined by the claims.

According to examples in accordance with an aspect of the invention, there is provided a processing arrangement comprising: an input/output; and one or more processors adapted to: receive at the input/output Terahertz (THz) sensing data for a body part of a subject; receive at the input/output movement or positioning data corresponding to the body part of the subject; determine perfusion information for the body part based upon a combination of the THz sensing data and the movement or positioning data; and generate a data output indicative of the determined perfusion information.

As discussed above, It is already established that THz frequency sensing can be used to derive tissue hydration information for a body part. However, hydration values alone are insufficient to perform differential diagnosis between different classes and localities of perfusion-related pathologies. Embodiments of the present invention are based on the concept of using a combination of THz sensing data and position information for the body part in question to facilitate more fine-grained analysis of a perfusion state of the body part. In particular, it is generally true that tissue hydration measurements (and the THz sensing data reflective thereof) vary as a function of pose of the body part, e.g. decreasing as the body part is elevated relative to the torso. In the case of a perfusion pathology, the hydration variation as a function of body part position or movement can be expected to differ compared to a healthy person, and thus the combination of THz data (whose values are indirectly reflective of hydration) and position information can provide a more rich source of information regarding the perfusion health status of an individual.

The positioning data could be position or movement data corresponding to the body part. It may be data from one or more inertial sensors such as an accelerometer and/or inertial measurement unit (IMU). It may be pose data. It may be data from a movement sensor.

In some embodiments, the body part maybe the leg. This may be one leg or both legs.

The one or more processors may further be adapted to: determine one or more tissue hydration measurements for the body part based on the THz data; and determine the perfusion information based upon the hydration measurements and the positioning data. Thus a combination of THz-derived hydration measurements and positioning information is used for deriving the perfusion information.

The processing arrangement may be adapted to determine the perfusion information based on THz sensing data captured for a plurality of different positions or poses of the body part. The positioning data is used to determine the position or pose that the body part is in, so that the captured THz sensing data can be married or correlated with body part position or pose. A data stream of THz sensing data samples may be acquired simultaneously with a positioning data stream.

The different positions or poses may correspond to different elevation angles of the body part. Elevation angles can be understood as elevation angle relative to horizontal or vertical, where horizontal is defined as orthogonal to the direction of gravity and vertical is defined as the direction of gravity. The relevant factor in this embodiment is increased gravitational pull along the length of the body part.

The processing arrangement may be adapted to determine a tissue hydration measurement for each of a plurality of detected positions or poses of the body part, and determine the perfusion information based upon the hydration measurements for the plurality of positions or poses of the body part. This embodiment is thus a combination of the features mentioned above. The different positions may be selected such that fluid drainage from the body part is different in each position. For example the different positions may be different elevation angles of the body part.

The processing arrangement may be adapted to determine the perfusion information based on a comparison between the tissue hydration measurements for the different body part positions or poses.

Time spent in a given pose also influences the measurable hydration. Hydration will change as a function of time. Thus, in one or more embodiments, the perfusion information may be further based upon a time duration for which the body part remained in each of the plurality of positions or poses. This may be a time duration that the body part remained in each of the plurality of positions or poses before the hydration measurement was taken. The time can for example be determined based on a time-stamp of each of the THz and positioning data samples in the received THz and positioning datasets, e.g. data streams.

The processing arrangement may be adapted to acquire THz data at two or more different time points during a time period that the body part remains in each of the different positions or poses. For example, it may acquire THz data and positioning data at recurrent series of time points over an examination period, and wherein the body part is moved between different poses over the course of the time period, the part remaining in each pose for a sub-portion of the overall examination period. This results in a data stream comprising positioning and THz data as a function of time for the multiple positions.

The processing arrangement may be adapted to receive THz sensing data for each of a plurality of sensing locations across the body part and to determine the perfusion information based on the data for the plurality of sensing locations.

In this embodiment, variation in hydration across the body part can be sensed and this can be used to derive more precise and detailed information about the perfusion state of the body part. It can provide more localized analysis of perfusion in the body part and/or can be used to further refine differential determination/diagnosis of perfusion pathologies.

The processing arrangement may be adapted to: determine a tissue hydration measurement for each of the plurality of sensing locations based on the THz sensing data for the plurality of sensing locations; perform a comparison between at least a subset of the tissue hydration measurements for the different sensing locations; and determine the perfusion information based in part on said comparison.

Differences between, or variation in, the hydration measurements for different areas of the body part can provide additional information about perfusion status. For instance, generally low hydration levels (low compared for example to a reference baseline level) which are low across the whole of the body part may be indicative of a different type of pathology to low hydration levels in only one localized area of the body part.

The processing arrangement may be adapted to differentially determine a class of perfusion pathology based on the comparison between the hydration measurements for the different measurement locations on the body part.

Thus, the processing arrangement enables differential diagnosis. This could be done based on use of a lookup table, or for example based on use of an artificial intelligence engine such as a machine learning algorithm.

The THz sensing data for the plurality of sensing locations across the body part may be data acquired from a plurality of THz sensors positioned at the plurality of sensing locations. Alternatively, the THz sensing data for the plurality of sensing locations across the body part may be data acquired from a single THz sensor sequentially moved between the plurality of locations.

In the first case, the THz sensing data comprises a plurality of temporally synchronous/simultaneous signal streams corresponding to the plurality of different physical sensors used to acquire the data. This is in comparison to the alternative approach, which would be to acquire the data using a single THz sensor which is moved sequentially across the plurality of different sensing locations. In this latter case, the THz sensing data would consist of a plurality of signal samples which are temporally asynchronous. The use of multiple sensors may be technically advantageous since it ensures that the measurements for the plurality of sensing locations all coincide with a common physical state of the body part at a particular moment in time. For example, if the body part is moved between different poses while measurements are taken, using the multiple sensors ensures that the measurement data for the multiple sensing positions corresponds to exactly the same set of pose positions.

The processing arrangement may be compatible with sensing data acquired in either setup, for example based on the processing arrangement comprising alternately selectable modes, each configured for receiving data in one of the two formats.

The processing arrangement may be adapted to: receive THz sensing data for a sequence of different elevation positions of the body part; determine a tissue hydration measurement for each of the elevation positions; and determine perfusion information based on fitting the determined sequence of hydration measurements to a pre-determined model function, the model function relating hydration measurement and elevation position.

Here, the processing arrangement is adapted to record hydration data as a function of elevation position and then determine the perfusion information based on the recorded relationship between the two. This is achieved more particularly by fitting the data to a predetermined model function, to derive function parameters which provide an indication of perfusion information.

In some embodiments, the perfusion information may be determined based on fitting the determined sequence of hydration measurements to a pre-determined model function, where the model function relates hydration measurement, elevation position and time. In other words, a data stream is received representing the elevation position as a function of time, and the hydration measurements may each be associated with a time stamp, and wherein the model is a function of elevation position and time. For example, the model may be a function of elevation position for each hydration measurement, as well as the time for which the body part remained in the elevation position before the relevant hydration measurement was taken, which may be determined from correlating the time stamp of the hydration measurement and the time stamps of the elevation position measurements.

The elevation position may be represented by a measure of an angle of inclination the body part relative to a starting angle, the angle determined based on the received positioning data. The positioning data may be data from an inertial sensor for example.

In accordance with one set of embodiments, the processing arrangement may be adapted to communicate with an actuation device adapted for implementing controlled adjustment in a position or pose of the body part, and the processing arrangement adapted to control sequential or continuous adjustment in the movement position, and wherein the positioning data is feedback signal data from the actuation device indicative of a current actuated position of the body part.

Here, position of the body part is actively controlled. This ensures enables acquisition of data for a consistent, standardized set of body part positions, which may improve reliability of confidence of the obtained perfusion information, and also improve comparability of results. It also eases the burden on an operator to manipulate the body part position manually.

By way of example, the feedback signal information may be feedback data from a servo motor of the actuation device. The positioning data may be data from an inertial sensor in some examples.

The determining of the perfusion information may in some embodiments be based on application of a machine learning algorithm. The machine learning algorithm may be stored in a local datastore for example, and retrieved during operation.

In alternative embodiments, the determining the perfusion information may be based on reference to a lookup table recording different perfusion status categories associated with each of a set of different possible measurement ranges for the THz sensing data and/or hydration measurement data.

In one set of embodiments, the processing arrangement may be further adapted to receive sensing data from one or more physiological parameter sensors. The perfusion information is determined based in part on the physiological parameter sensing data.

In some embodiments, the processing arrangement may be adapted to determine a differential diagnosis between a perfusion pathology and one or more further non-perfusion pathologies based on the physiological parameter data.

According to this embodiment, the system is operable to determine not only perfusion pathologies, but other systematic pathologies due to the inclusion of additional physiological parameter information such as heart rate information or other hemodynamic parameters, which allow for differential diagnosis between perfusion pathologies and other more general systematic pathologies.

In accordance with one or more embodiments, the processing arrangement is adapted to determine information relating to status of one or more perfusion compensatory mechanisms, such as pulse rate and pulse volume response following a change in inclination angle of the body part. After changing the elevation angle of a body part, such as the leg, this leads to transfer of fluid from the body part to the central circulation (or vice versa), resulting in an initial drop (or rise) in tissue perfusion. Following this, one or more compensatory mechanisms are triggered such as an increase in pulse rate and pulse volume to maintain tissue perfusion at a desired level. An adequacy status of the compensatory mechanisms can be determined based on monitoring changes in one or more physiological parameters, such as pulse rate and pulse volume, following a change in body part position.

A further aspect of the invention also provides a system comprising:

a THz sensing apparatus for acquiring THz sensing data for a body part;
a body part position or movement data source for use in sensing changes in a position of the body part; and
a processing arrangement, comprising one or more processors adapted to determine perfusion information for the body part based upon data from the THz sensing apparatus and based upon the body part position or movement data.

The body part position or movement data source may be a position or movement sensor, such as an inertial sensor, adapted to attach to the body part of the subject. Alternatively, it may be an output from an actuation device (as described above) which is adapted to controllably and mechanically move the body part through a series of controlled positions, in which case the source of the movement data may be feedback data from the actuator, indicative of actuation position.

A further aspect of the invention also provides a method, comprising: receiving Terahertz (THz) sensing data for a body part of a subject; receiving positioning data corresponding to the body part of the subject; determining perfusion information for the body part based upon a combination of the THz sensing data and the positioning data; and generating a data output indicative of the determined perfusion information.

A further aspect of the invention also provides a computer program product comprising computer program code, the computer program code being executable on a processor, wherein the code is configured to cause the processor to perform a method in accordance with any example or embodiment outlined above or described below, or in accordance with any claim of this application.

The code may be configured to cause the processor to perform the method when the processor is operatively coupled with a THz sensing apparatus for acquiring THz sensing data for a body part, and a body part position or movement data source.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically depicts components of an example system comprising a processing arrangement according to one or more embodiments;
Fig. 2 illustrates movement of a body part between different poses as part of an example measurement operation according to one or more embodiments;
Fig. 3 illustrates acquired hydration and body part position data as a function of time over an examination period;
Fig. 4 shows an example decision tree illustrating categorization of perfusion pathologies based on hydration data and body part positioning data; and
Fig. 5 shows an example decision tree illustrating differential diagnosis of perfusion pathologies based on hydration data and body part positioning data.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means for determining perfusion information relating to a body part of a subject based on THz data relating to the body part acquired over the course of an examination period, during which the body part is placed in a number of different positions or poses, and based on positioning or movement information relating to the body part during that examination period.

Lower extremities are a common site for various perfusion related abnormalities. These pathologies are usually diagnosed based on clinical history and physical examination, which are subjective in nature. Moreover, no beside method is available for a GP for a quick screening, differential diagnosis and grading of perfusion abnormalities.

The current methods of diagnosis of musculoskeletal pathologies in particular have at least the following limitations: subjectivity in assessment of perfusion changes; non-availability of a bedside method for quick diagnosis of different perfusion abnormalities; and non-availability of a bedside method for quick grading of different perfusion abnormalities.

Embodiments of the present invention propose use of Terahertz spectroscopy (THz) in combination with positioning or movement data relating to the patient's body part (for example a sensor such as an accelerometer or an inertial measurement unit (IMU)). Optionally, physiological parameter data may additionally be acquired (e.g., one or more of heart rate, pulse volume, blood pressure, temperature), and the perfusion information determined in part based on this data. Changes in tissue hydration with change in body part positions may be determined. The acquired data signals may be co-analyzed for assessment, quantification, and optionally differential diagnosis of, perfusion abnormalities. In some examples, the data can be used to classify different types of abnormality.

In some embodiments, additionally information about clinical history, and/or physiological parameter data is taken into account in determining the perfusion information.

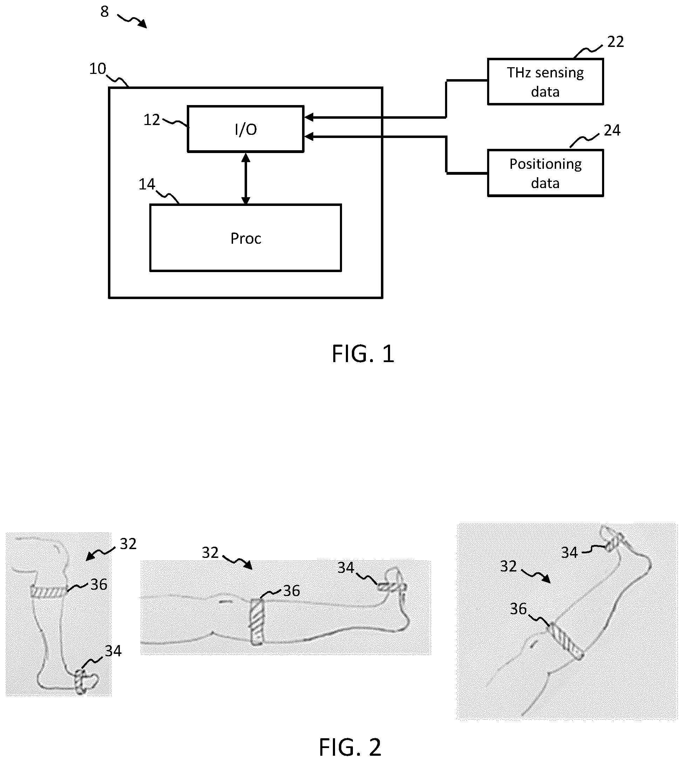

Fig. 1 schematically illustrates components of an example system 8 according to one or more embodiments. The system comprises a processing arrangement 10. One aspect of the invention provides the processing arrangement alone. Another aspect of the invention provides the entire system 8.

The processing arrangement 8 comprises an input/output 12 which is communicatively coupled with one or more processors 14. The one or more processors are configured to perform a computer-implemented method or routine, the steps of which will now be outlined.

The one or more processors 14 are adapted receive at the input/output 12 Terahertz (THz) sensing data for a body part of a subject. The one or more processors are further adapted to receive at the input/output positioning data corresponding to the body part of the subject. The one or more processors are further adapted to determine perfusion information for the body part based upon a combination of the THz sensing data and the positioning data. The one or more processors are further adapted to generate a data output indicative of the determined perfusion information.

The illustrated system of Fig. 1 comprises a terahertz sensing data source 22, and a positioning data source 24 adapted to provide respectively the Terahertz sensing data relating to the body part of the subject, and the position or movement data relating to the position or movement of the body part occurring throughout the examination period over which the Terahertz sensing data is acquired. These data sources may take different forms, as will be discussed further below. For example, the positioning data source could be an arrangement of one or more position or movement or inertial sensors, and/or may be a data output from a motor or actuator system arranged for mechanically moving the body part to controllable positions.

The data output generated by the processing arrangement may be simply a data representation of the derived perfusion information. This may for example be output to a datastore for future retrieval and reference, or to a further computer or server. In some examples it may additionally or alternatively comprise a control output for controlling a user interface to generate a sensory output indicative of the derived perfusion information. For example, the sensory output may comprise a visual display representative of the derived information, or an acoustic output representative of the derived information. In some examples, the system 8 may comprise a display unit adapted to display the derived perfusion information, for example in real-time.

The THz sensing data may be received from a THz sensing apparatus. The system may comprise a THz sensing apparatus. This may comprise one or a plurality of THz sensing elements, each operable to emit/transmit THz frequency electromagnetic radiation and to receive or detect radiation reflected back from the body responsive to the applied THz signals.

The THz sensing apparatus may comprise one or more THz sensing probes or units, each comprising one or a plurality of THz sensing elements as mentioned above. By way of example, the THz sensing apparatus may comprise a portable handheld unit (e.g. probe) having a housing and comprising one or more integrated THz elements. In addition or instead, the THz sensing apparatus may comprise one or more patches, removably adherable to the skin of a subject, and incorporating one or more THz sensing elements having an electromagnetic (EM) output area arranged so as to face in toward the skin/body when the patch is applied to the skin.

In some examples, a position or movement sensor may be integrated in the THz sensing unit or probe, e.g. a handheld device with integrated position or movement sensor.

The THz sensing unit may have a controllable spectral window for the radiation output. This may be fixed in advance, or may be adjustable by one or more processors comprised by the system. For example, a spectral window which may be used according to one or more specific embodiments is 0.4-0.7 THz or 0.2 THz to 0.9 THz.

In use, the THz sensing unit or probe is applied to the body part of the subject. The body part may be an extremity such as the leg or arm. It may be applied to one or more of the extremities.

The THz sensing data may be used to obtain hydration information from the tissue according to the equations and models described earlier in this disclosure.

For measurement, a single THz sensor may be used, for application to a single sensing location on the body at any one time, or a combination of THz sensing units may be used, to thereby obtain measurements from a plurality of different sensing locations on the body part at once, to create a tomographic reading for the tissue. Alternatively, a single THz sensing unit can also be used to obtain a plurality of sequential measurements at a sequence of different sensing locations on the body to create a tomographic reading. A tomographic reading means a measurement dataset which comprises measurement data representative of a 2D or 3D spatial region of the body part, comprising a plurality of measurement samples at different locations across the spatial region. The measurement samples may be hydration measurements, or may be the THz sensing signal measurements.

The positioning data could be position or movement data corresponding to the body part. It could be data from an inertial sensor permitting a pose of the body part to be determined, for example an angle of inclination. It could be data from a movement sensor such as an accelerometer, permitting changes in position to be derived from acceleration data. The positioning data could be pose data for the body part. The data may be received from a movement sensor such as an IMU.

Optionally, a temperature sensor may be used to acquire a local surface temperature of the tissue at the sensing location at which the THz sensing unit is positioned. This may be integrated in the THz sensing unit, or may be a separate unit.

In operation, THz sensing data is acquired for multiple poses or positions of the body part. For example, the patient is asked to lift the body part up from a starting position through a series of different elevation angles. THz sensing data is acquired and may be used to derive tissue hydration information at the different positions. A position or movement sensor, which may be coupled to the body part, such as an IMU sensor, may be used to derive the positioning information for the body part over time. All of these signals may then be coanalyzed to derive information about perfusion abnormalities. The signals may be temporally correlated to one another.

A number of example embodiments will now be outlined in more detail.

According to one set of embodiments, a THz sensing unit is used to acquire THz sensing data at a single location on a body part (e.g. an extremity) as the body part is moved through a series of poses. This embodiment permits broad categorization of perfusion pathologies relating to the body part.

The THz sensing apparatus comprises a THz sensing unit or device comprising one or a plurality of integrated THz sensing elements as described above. This may be a probe unit, or a body-mountable or wearable sensor unit such as a wearable band or patch. A position or movement sensor is coupled to the body part of the subject adapted to generate data for deriving position or pose of the body part over time. The position or movement sensor may be integrated within the THz sensing unit, or may be a separate sensing unit mounted separately to the body part. The position or movement sensor may for example comprise an accelerometer and/or an IMU.

To perform the examination, the subject is asked to place their body part in a starting position. For example, the patient is asked lie down flat on a surface such as a bed, or to sit in a chair. Depending on the patient's position, the device is applied to a location on the body part (e.g. leg or arm) over a particular anatomical area. This may be an area which is prone to fluid accumulation (e.g., the dorsal surface of the foot, or the area just above ankle joint).

The patient is asked to move the body part to a new position. This may be a position more elevated than the starting position, i.e. holding the body part at a higher elevation angle than the starting position. Elevation angle can be understood as elevation angle relative to horizontal, where horizontal is defined as orthogonal to the direction of gravity. Thus, to elevate the body part, one end of the body part is raised in a vertical direction relative to the other (vertical defined by a direction of gravity). For example a leg or arm is lifted from a resting position, up to a more elevated position.

The patient is asked to hold the body part in the new position for a defined period of time, or a defined minimum period of time, so as to allow gravity-assisted drainage of fluid from the body part. The movement of the body part can be done voluntarily by the subject, or manually by a clinician, or electronically using a mechanical actuation device in a controlled manner.

By way of illustration, Fig. 2 shows the movement of the body part 32 between (a) a starting position, (b) a second position, and (c) a third position, where the second position has the body part (leg in this example) at a higher elevation angle relative to gravitational vertical than the starting position, and the third position has the body part at a higher elevation angle that the second position. Fig. 2 illustrates the THz sensing unit 34, which in this case takes the form or a body-mountable THz sensing unit, and further illustrates the position or movement sensor 36, also taking the form of a body-mounting unit with a position or movement sensor integrated within a housing thereof.

The position or movement sensor 36 generates a positioning data output which is communicated to the processing arrangement 10 as described above. This positioning data is user to detect and quantify the body part movements. For example, an inertial sensor such as an accelerometer or IMU can be used to detect and quantify changes in elevation angle of the body part to which it is fixedly mounted.

Optionally, the positioning data output may also be used to trigger activation of the terahertz sensor unit 34 (or trigger activation of data acquisition using the terahertz sensor unit) responsive to detecting that the body part has been moved to one of a predetermined set of measurement positions or poses for the body part, or responsive to detecting that the body part position has been changed by a predetermined amount, for example the body part has been elevated by a certain elevation angle compared to the previous position or pose in which it was placed or which terahertz sensing data was acquired.

THz sensing data is acquired at each different position or pose in which the body part is held. The processing arrangement 10 is adapted to derive from the THz data, hydration measurement data for each of the positions or poses. For example, a hydration reading may be obtained before, during and after the leg movement. Hydration measurements may be obtained recurrently, at regular intervals (i.e. continuously or intermittently) throughout the examination period. The change in hydration as a function of body part position can be determined. This may be used to derive perfusion information relating to the body part. For example, this may be used to detect perfusion abnormalities, as will be described in greater detail to follow.

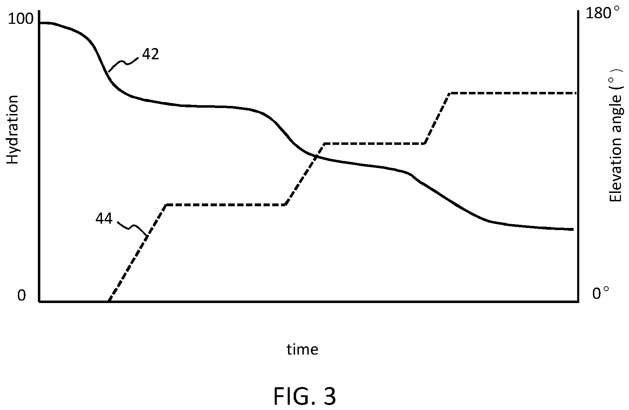

By way of illustration, Fig. 3 shows example hydration measurement data 42 and body part position data 44 as a function of time over the course of an examination of a subject. The hydration measurement data 42 comprises a series of measurement samples which may be acquired at regular temporal intervals or may be acquired at each different body part position. Each hydration measurement is based on THz sensing data acquired at the corresponding time point. In the illustrated example, the positioning data 44 corresponds to body part elevation angle relative to gravitational vertical, or relative to horizontal. The positioning data stream may comprise a series of data samples acquired at regular intervals throughout the examination. By temporally correlating or registering the two datasets 42, 44 to one another, the hydration as a function of body part position can be determined. In addition, since each hydration measurement sample and position measurement sample is time-stamped this also allows for assessment of hydration as a function of both body part position and time. As explained above, the length of time in which a body part remains in a given position also has an effect on the measurable hydration, since more fluid drains from the body part as more time elapses.

Although in the above descriptions, reference has been made to deriving hydration measurements for each position of the body part, this is not essential. For example, instead of explicitly computing hydration measurements based on acquired Terahertz sensing data, instead the terahertz sensing data might be directly related to perfusion information for example by means of a suitable lookup table or by means of a suitable algorithm, such as a regression algorithm or machine learning algorithm.

As mentioned, the derived hydration information in relation to the body part position can be used to categorize different types of perfusion pathology which may exist in the body part. In operation, the categorization of the perfusion pathology may be based on use of a lookup table or one or more algorithms as will be discussed further below.

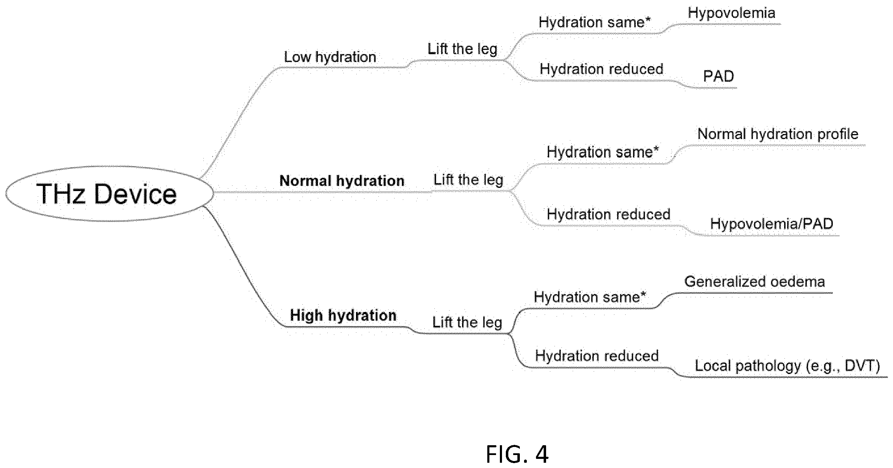

The categorization may be understood as following a decision tree structure, based on the hydration measurements and body part positioning data. To illustrate, Fig. 4 shows a schematic decision tree illustrating how categorization of perfusion pathologies may be performed based on acquired data. An example process of categorizing perfusion pathologies will now be described.

Perfusion pathologies may be categorized based on an initial hydration measurement of tissue (with the body part in a starting position, before body part movement), and based on subsequent changes in the measurable hydration following movement to a new position.

The initial hydration of tissue may be compared with one or more standard or reference hydration values. The standard hydration values may be stored in a local or remote datastore for example. They may be stored in a database for example. The reference hydration values may be values derived from population-based studies or from the literature. In some examples, they may be at least partially personalized to the subject, for example based on one or more demographic or health - related indicators.

Based on the comparison with the one or more reference hydration values, initial tissue hydration may be categorized into one of three broad categories - (1) normal hydration; (2) low hydration; and (3) high hydration. This is illustrated in Fig. 4.

During the examination, the position of the body part is moved from the initial position to a new position. The tissue hydration after movement is then compared with tissue hydration before movement.

By way of example, the increased elevation of the body part, such as the leg, leads to transfer of fluid from the body part to the central circulation, resulting in an initial drop in tissue perfusion. This requires the heart to pump against gravity to maintain perfusion. This will be followed by compensatory mechanisms such as an increase in pulse rate and pulse volume to maintain tissue perfusion at a desired level. Pulse rate and pulse volume may in some examples be acquired simultaneously with the Terahertz sensing data and the positioning data to further aid categorization, but this is not essential.

In the case of normal hydration when the body part is in the starting position, a significant drop in hydration (for example below a predefined threshold, which may for example be predetermined from population-based studies or literature) after the body part movement indicates inadequacy of compensatory mechanisms. This could due to a local pathology such as peripheral arterial disease (PAD) or due to a systematic pathology such as hypovolemia/dehydration. This is illustrated by the middle branch of the decision tree in Fig. 4.

In the case of initial low hydration when the body part is in the starting position, this could be an indication of either PAD or hypovolemia. In such cases, a significant drop in hydration (for example below a predefined threshold, which may for example be predetermined from population-based studies or literature) after movement of the body part strongly indicates PAD. This is illustrated by the branch of the decision tree of Fig. 4.

In the case of initial high hydration when the body part is in the starting position, this could be an indication of abnormal collection of fluid in the tissue. This could be either due to a local pathology such as deep vein thrombosis or varicose vain, or could be due to generalized oedema. In such cases, a significant drop in hydration (for example below a predefined threshold, which may for example be predetermined from population-based studies or literature) after movement of the body part indicates local pathology. This is illustrated by the lower branch of the decision tree of Fig. 4.

One or more algorithms may be locally stored by the processing arrangement and used during operation to derive the perfusion information, where the perfusion information comprises a categorization of the perfusion pathology (if such pathology is present). The one or more algorithms may comprise by way of non-limiting example one or more of: a decision tree, logistic regression, support vector machine, random forests, and neural networks. The one or more algorithms may be trained on a large database of training data which comprises THz measurement data before and after patient movements along with positioning data and time information (as discussed above), each training data entry being manually tagged according to the particular perfusion pathology which was present in the subject from which the data was acquired.

Although the above embodiment was illustrated with reference to the leg being the body part, the embodiment is applicable to any body part. The body part may be an extremity. The particular body part is not inextricably tied to the other details of the embodiment.

According to a further set of embodiments, THz sensing data may be acquired for a plurality of locations on a body part (e.g. an extremity), or multiple body parts, as the body part(s) is moved through a series of poses. This embodiment permits differential diagnosis and categorization of the perfusion abnormalities. The steps according to this set of embodiments will now be outlined.

As in the preceding embodiment, a THz sensing unit comprising one or more THz sensing elements may be used, where this may be a handheld probe device or a body-mountable unit such as band or patch. A positioning sensor is also provided, such as an IMU or accelerometer, and this may be integrated in the THz sensor or may be provided separately. In this set of embodiments, THz sensing data is acquired for a plurality of measurement locations. Thus, in some examples, a plurality of THz sensing units may be employed, each for positioning at a different location on the body part or parts. In this way, THz data can be acquired for the plurality of sensing locations simultaneously.

Instead of multiple units, a single unit having an array of THz sensing elements in a particular geometrical arrangement may be provided, for example a patch which provides, when mounted to the body part, THz sensing elements positioned at an array of different locations. In further examples, a single THz sensing unit may be used, but wherein the sensing data for the multiple sensing locations is acquired sequentially, by moving the single sensing unit between the locations. The different sensing locations may be different locations on the same body part (which is suspected as having a pathology) and/or may be locations on more than one body part, e.g. the opposite of the pair of extremities, e.g. the other arm, leg, foot, hand.

To perform the examination, the patient is asked to position the body part (e.g. leg) in a starting position. For example the patient is asked to lie down flat on a surface or sit in a chair. Depending upon the patient's position, the one or more THz sensor units are applied to the patient's body part over one or more anatomical areas of interest, e.g. an area which is prone to fluid accumulation (e.g., the dorsal surface of foot, or the area just above ankle joint).

THz sensing data is acquired for the multiple sensing locations. The processing arrangement 10 derives tissue hydration measurements for each of the multiple sensing locations. These may be compared with each other in some examples. Comparison of tissue hydration at multiple locations on a given body part (e.g. for the leg, anterior vs. posterior; upper vs lower), or for a pair of body parts (e.g. left vs right) can be used to quantify changes in perfusion, as will be further described below.

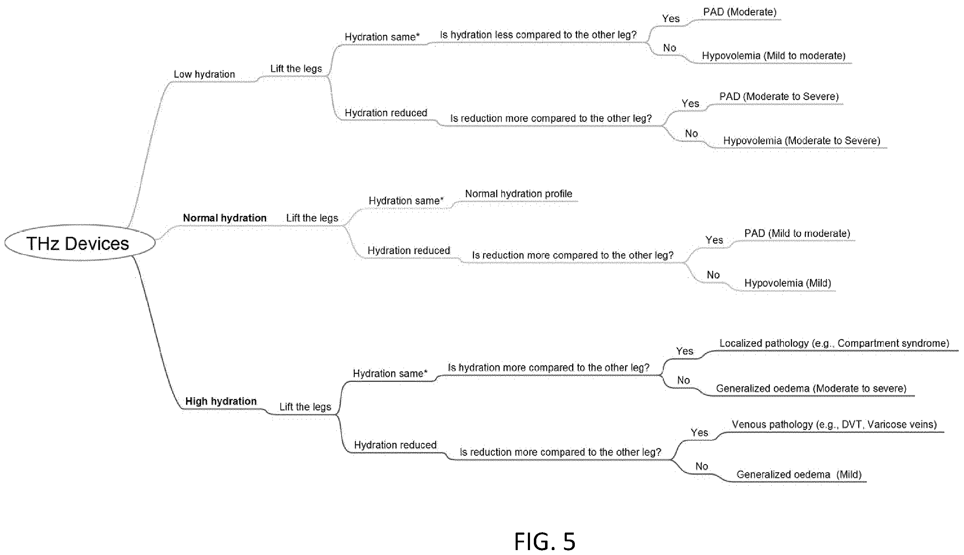

There will now be described an example implementation of this set of embodiments for differential diagnosis of perfusion pathologies. This is based on a scenario in which THz sensing data, and corresponding hydration measurements, were acquired for two sensing locations on each of the left and right extremity, e.g. left and right leg. To illustrate, Fig. 5 shows a schematic decision tree illustrating how differential diagnosis of perfusion pathologies may be performed based on the acquired data.

As described for the previous set of embodiments, an initial hydration measurement is acquired, this time at each measurement location (with the body part in a starting position, before body part movement).

The initial hydration of tissue may be compared with one or more standard or reference hydration values. The standard hydration values may be stored in a local or remote datastore for example. The reference hydration values may be values derived from population-based studies or from the literature. In some examples, they may be at least partially personalized to the subject.

Based on the comparison with the one or more reference hydration values, initial tissue hydration may be categorized into one of three broad categories - (1) normal hydration; (2) low hydration; and (3) high hydration. This is illustrated in Fig. 5.

During the examination, the position of the body parts is moved from the initial position to a new position for each. The tissue hydration after movement at each measurement location is then compared with the initial tissue hydration before movement. The hydration measurements from the plurality of measurement locations are also compared with one another.

For the purposes of this examples, a change in tissue hydration after the movement will be classified as "Same hydration" (Fig. 5) if it is within a certain pre-defined threshold (e.g. based pre-determined based on population-based studies or literature) of the initial hydration measurement. If the subsequent hydration measurement is less than the initial value by a pre-defined threshold, then it is categorized as "Reduced hydration" (Fig. 5).

With reference to Fig. 5, the information relating to change in tissue hydration before and after movement may be used to differentially classify perfusion abnormalities.

For example, in case of a generalized pathology (e.g. oedema due to congestive cardiac failure) the hydration profile of both the extremities will be affected similarly; whereas in case of localized pathology (e.g. DVT, bone fracture) the two extremities will have different hydration profiles. This comparative information can thus be used to differentiate and as well as to grade the perfusion abnormalities.

One or more algorithms may be locally stored by the processing arrangement and used during operation to derive the perfusion information, where the perfusion information comprises a differential classification or diagnosis of the perfusion pathology (if such pathology is present). The one or more algorithms may comprise, by way of non-limiting example, one or more of: a decision tree, logistic regression, support vector machine, random forests, and neural networks. The one or more algorithms may be trained on a large database of training data which comprises THz measurement data before and after patient movements, each training data entry being manually tagged according to the particular perfusion pathology which was present in the subject from which the data was acquired.

According to one or more embodiments, grading of perfusion pathologies can be determined. One example set of embodiments operable to derive grading of perfusion pathologies will now be outlined. This embodiment employs use of controlled movement of the body part using a mechanical actuation system, but this is not essential.

To perform the examination, the subject is asked to place their body part in a starting position. For example, the patient is asked lie down flat on a surface such as a bed, or to sit in a chair. Depending on the patient's position, the THz sensing unit is applied to a location on the body part (e.g. leg or arm) over a particular anatomical area. This may be an area which is prone to fluid accumulation (e.g. the dorsal surface of the foot, or the area just above ankle joint).

An actuation device is mechanically coupled or engaged with the body part, where the actuation device is adapted for implementing controlled adjustment of a position or pose of the body part. The processing arrangement 10 is adapted to control sequential (e.g. stepwise) or continuous adjustment in the movement position through a series of different poses.

In this example, the positioning data supplied to the processing arrangement may be feedback signal data from the actuation device indicative of a current actuated position of the body part. Alternatively, a dedicated position or movement sensor may be coupled to the body part, e.g. an accelerometer or IMU, wherein this sensor is used to detect degree of the leg movements. It may optionally also be used to activate THz sensor data acquisition when a desired degree of patient movement is obtained.

By way of example, the subject's body part (e.g. leg) is moved in a stepwise manner using the mechanical actuation device. In each step, the subject's body part is lifted by a certain amount so as to change the elevation angle by a pre-defined angular interval (e.g., 30°). The body part is then held in the relevant position for a pre-defined amount of time.

THz sensor data is obtained before, during and after the leg movement. For example, a series of data samples may be acquired continuously or intermittently throughout the examination. A hydration measurement may thus be determined for each different body part elevation position. This thereby results in a sequence of hydration measurements for a corresponding sequence of different elevational positions. More than one measurement may be obtained for each body part position in some examples.

In advantageous examples, the perfusion information may be determined based on fitting the determined sequence of hydration measurements to a pre-determined model function, the model function relating hydration measurement and elevation position. It may in some cases relate hydration measurement to elevation position and time. Time taken by fluid to gravitate can provide information about its nature (e.g. transudate vs exudate).

The elevation position may be represented by a measure of an angle of inclination the body part relative to a starting angle, the angle determined based on the received positioning data.

By way of example, a relationship between change in tissue hydration, corresponding change in angle, and time of leg movement can be expressed mathematically as:

$$\Delta H = f(\text{IH, AM, T, and PP}) \qquad (4)$$

where ΔH = Change in tissue hydration after movement,

IH = Initial tissue hydration before leg movement,

AM = Angle of the leg movement,

T = Time spend in a particular position.

PP = Physiological parameters (e.g. heart rate, pulse volume, blood pressure)

The function *f* is a pre-determined model function which may be stored in a local or remote datastore.

Based on an acquired data set of tissue hydration measurements and body part angular elevations, each as a function of time, it is possible, by fitting the data to the model function, to derive an expected change in tissue hydration (ΔH) for any given value of IH, AM, T and PP for different types and grades of perfusion abnormalities.

The resulting fitted model function may be referred to as a hydration profile, and it relates tissue hydration, body part angle of elevation and time. For a given body part, the hydration profiles at each point in the function space (i.e. degree of a leg movement and time spent in a particular position) may be used to grade perfusion abnormalities. One example for this is provided as follows.

A reduction in tissue hydration (below a predefined threshold) following an elevation angle increase, while the absolute elevation angle remains below a defined threshold, and which occurs following a short duration of time, indicates a high grade of perfusion abnormality (e.g., severe PAD or peripheral arterial blockage of greater than a defined threshold percentage). By contrast, an observed reduction in tissue hydration (below a predefined threshold) following increase in angle, while absolute angle is above a defined threshold (i.e. at higher angles) may indicate a strong compensatory mechanism, and a low grade of perfusion abnormality.

In some examples, instead of explicitly fitting a pre-defined model function, the grading of the one or more abnormalities may be based on application of one or more pre-defined algorithms, The one or more algorithms may comprise by way of non-limiting example one or more of: a decision tree, logistic regression, support vector machine, random forests, and neural networks. The one or more algorithms may be trained on a database of training data which comprises training data entries, each comprising THz measurement data or hydration measurement data for a set of different body part elevation angles, each data point tagged with a time-stamp, and wherein each training data entry is manually tagged according to the particular grade of perfusion pathology which was present in the subject from which the data was acquired.

According to one or more embodiments, perfusion information obtained using the system can be used to detect and assess one or more pathologies or abnormalities of other physiological systems. This can be achieved in some examples through additional acquisition of physiological parameter data such as heart rate, blood pressure and/or pulse volume.

For example, heart rate and blood pressure are important parameters for regulation of tissue perfusion. An inclination of the leg leads to transfer of fluid from the leg to the central circulation and this results in an initial drop in tissue perfusion, since the heart is required to pump against gravity to maintain perfusion. Following the drop in perfusion, compensatory mechanisms are engaged to counteract the drop, such as increase in pulse rate, pulse volume and blood pressure to maintain tissue perfusion at a desired level.

According to an example set of embodiments, physiological parameters such as pulse rate, pulse volume, and/or blood pressure are monitored during the examination of the subject and the acquisition of the THz sensing data. The physiological parameter monitoring can be achieved through any suitable means, for example a PPG sensor, ECG sensing, or by cardiac ultrasound (e.g. Doppler) sensing. The one or more parameters are monitored simultaneously with the acquisition of the THz sensing data at the plurality of body part positions.

From Equation 4 (see above), it is known that a change in tissue hydration ($\Delta$H) is also a function of physiological parameter(s). Therefore, Equation 4, may be modified as:

$$\Delta PP = f(\Delta H, IH, AM, T, \text{and } iPP) \tag{5}$$

where $\Delta$PP = Change in physiological parameters (e.g., blood pressure)
$\Delta$ H = Change in tissue hydration after movement,
IH = Initial tissue hydration before leg movement,
AM = Angle of the leg movement,
T = Time spend in a particular position.
iPP = Initial value of physiological parameters (e.g., blood pressure)

The function $f$ may be a further pre-defined model function. This may be stored on a local or remote datastore. The model function may be fitted to the obtained sensing data, in particular the hydration measurement data, the time data, the movement or position data and the physiological parameter data. Once the model function is fitted, this may permit derivation of expected change in physiological parameters for any given value of $\Delta$H, IH, AM, T, and iPP for different types and grades of cardiac abnormalities.

Therefore, the obtained data related to changes in a physiological parameters during the movement of the body part (e.g. leg) along with the tissue hydration information during the same movements can be used for not only quantification and differential diagnosis of perfusion abnormalities but also to judge adequacy or inadequacy or compensatory mechanisms (e.g., severity of heart failure) and related pathologies.

A further aspect of the invention provides a system comprising: a THz sensing apparatus for acquiring THz sensing data for a body part; a body part position or movement data source for use in sensing changes in a position of the body part; a processing arrangement, comprising one or more processors adapted to determine perfusion information for the body part based upon data from the THz sensing apparatus and based upon the body part position or movement data.

Implementation options and details for each of the above features may be understood and interpreted in accordance with the explanations and descriptions provided above.

A further aspect of the invention provides a method, comprising: receiving Terahertz (THz) sensing data for a body part of a subject; receiving positioning data corresponding to the body part of the subject; a determining perfusion information for the body part based upon a combination of the THz sensing data and the positioning data; and generating a data output indicative of the determined perfusion information.

Implementation options and details for each of the above steps may be understood and interpreted in accordance with the explanations and descriptions provided above for the apparatus aspect of the present invention. Any of the examples, options or embodiment features or details described above may be applied or combined or incorporated into the method aspect of the invention.

A further aspect of the invention provides a computer program product comprising computer program code, the computer program code being executable on a processor, wherein the code is configured to cause the processor to perform a method in accordance with any example or embodiment outlined above or described below, or in accordance with any claim of this application.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". Any reference signs in the claims should not be construed as limiting the scope.

**Claims**

1. A processing arrangement comprising:

    an input/output; and
    one or more processors adapted to:

        receive at the input/output Terahertz (THz) sensing data for a body part of a subject;
        receive at the input/output positioning data corresponding to the body part of the subject;
        determine perfusion information for the body part based upon a combination of the THz sensing data and the positioning data; and
        generate a data output indicative of the determined perfusion information.

2. A processing arrangement as claimed in claim 1, the one or more processors further adapted to:

    determine one or more tissue hydration measurements for the body part based on the THz data; and
    determine the perfusion information based upon the hydration measurements and the positioning data.

3. A processing arrangement as claimed in claim 1 or 2, wherein the processing arrangement is adapted to determine the perfusion information based on THz sensing data captured for a plurality of different positions or poses of the body part, and optionally wherein the different positions or poses are different elevation angles of the body part.

4. A processing arrangement as claimed in claim 3, wherein the processing arrangement is adapted to determine a tissue hydration measurement for each of a plurality of detected positions or poses of the body part, and determine the perfusion information based upon the hydration measurements for the plurality of positions or poses of the body part.

5. A processing arrangement as claimed in claim 4, wherein the processing arrangement is adapted to determine the perfusion information based on a comparison between the tissue hydration measurements for the different body part positions or poses.

6. A processing arrangement as claimed in claim 4 or 5, wherein the perfusion information is further based upon a time duration for which the body part remained in each of the plurality of positions or poses.

7. A processing arrangement as claimed in any of claims 1-6, wherein the processing arrangement is adapted to receive THz sensing data for each of a plurality of sensing locations across the body part and to determine the perfusion information based on the data for the plurality of sensing locations.

8. A processing arrangement as claimed in claim 7, wherein the processing arrangement is adapted to:

   determine a tissue hydration measurement for each of the plurality of sensing locations based on the THz sensing data for the plurality of sensing locations;
   perform a comparison between at least a subset of the tissue hydration measurements for the different sensing locations; and
   determine the perfusion information based in part on said comparison.

9. A processing arrangement as claimed in claim 7 or 8, wherein:

   the THz sensing data for the plurality of sensing locations across the body part is data acquired from a plurality of THz sensors positioned at the plurality of sensing locations; or
   data acquired from a single THz sensor sequentially moved between the plurality of locations.

10. A processing arrangement as claimed in any of claims 1-9,
    wherein the processing arrangement is adapted to:

    receive THz sensing data for a sequence of different elevation positions of the body part;
    determine a tissue hydration measurement for each of the elevation positions; and
    determine perfusion information based on fitting the determined sequence of hydration measurements to a pre-determined model function, the model function relating hydration measurement and elevation position, and

    optionally wherein the elevation position is represented by a measure of an angle of inclination the body part relative to a starting angle, the angle determined based on the received positioning data.

11. A processing arrangement as claimed in any of claims 1-10, wherein the processing arrangement is adapted to communicate with an actuation device adapted for implementing controlled adjustment in a position or pose of the body part, and the processing arrangement adapted to control sequential or continuous adjustment in the movement position, and wherein the positioning data is feedback signal data from the actuation device indicative of a current actuated position of the body part.

12. A processing arrangement as claimed in any of claims 1-11, wherein the processing arrangement is further adapted to receive sensing data from one or more physiological parameter sensors, and
    wherein the perfusion information is determined based in part on the physiological parameter sensing data; and/or
    wherein the processing arrangement is adapted to perform differential diagnosis between a perfusion pathology and one or more further non-perfusion pathologies based on the physiological parameter data.

13. A system comprising:

    a THz sensing apparatus for acquiring THz sensing data for a body part;
    a body part position or movement data source for use in sensing changes in a position of the body part;
    a processing arrangement, comprising one or more processors adapted to determine perfusion information for the body part based upon data from the THz sensing apparatus and based upon the body part position or movement data.

14. A method, comprising:

receiving Terahertz (THz) sensing data for a body part of a subject;
receiving positioning data corresponding to the body part of the subject;
determining perfusion information for the body part based upon a combination of the THz sensing data and the positioning data; and
generating a data output indicative of the determined perfusion information.

15. A computer program product comprising computer program code, the computer program code being executable on a processor, wherein the code is configured to cause the processor to perform a method in accordance with claim 14.

FIG. 1

FIG. 2

FIG. 3

FIG. 4

FIG. 5

Europäisches
Patentamt
European
Patent Office
Office européen
des brevets

# EUROPEAN SEARCH REPORT

Application Number

EP 21 16 7783

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/039326 A1 (CRABTREEE VINCENT PETER [GB] ET AL) 6 February 2014 (2014-02-06) <br> * figures 1-3 * <br> * paragraphs [0026] - [0064] * <br> ----- | 1-15 | INV. <br> A61B5/026 <br> A61B5/0507 <br> A61B5/00 <br> A61B5/11 |
| A | HERNANDEZ-CARDOSO GORETTI G ET AL: "Development of a method of evaluation of diabetic foot deterioration by terahertz spectroscopic image", 2017 42ND INTERNATIONAL CONFERENCE ON INFRARED, MILLIMETER, AND TERAHERTZ WAVES (IRMMW-THZ), IEEE, 27 August 2017 (2017-08-27), pages 1-2, XP033164972, DOI: 10.1109/IRMMW-THZ.2017.8066861 [retrieved on 2017-10-12] * the whole document * ----- | 1-15 | |

TECHNICAL FIELDS
SEARCHED (IPC)

A61B

The present search report has been drawn up for all claims

| Place of search | Date of completion of the search | Examiner |
|---|---|---|
| The Hague | 28 September 2021 | Dhervé, Gwenaëlle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 7783

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2021

| Patent document cited in search report | Publication date | Patent family member(s) | Publication date |
|---|---|---|---|
| US 2014039326 A1 | 06-02-2014 | AT 440538 T | 15-09-2009 |
| | | AU 2005203912 A1 | 21-07-2005 |
| | | AU 2011201021 A1 | 31-03-2011 |
| | | BR PI0506752 A | 22-05-2007 |
| | | CA 2552924 A1 | 21-07-2005 |
| | | CN 1909827 A | 07-02-2007 |
| | | CN 101732045 A | 16-06-2010 |
| | | CY 1109638 T1 | 13-08-2014 |
| | | DK 1694203 T3 | 07-12-2009 |
| | | EP 1694203 A1 | 30-08-2006 |
| | | EP 2108309 A1 | 14-10-2009 |
| | | EP 2108310 A1 | 14-10-2009 |
| | | ES 2331617 T3 | 11-01-2010 |
| | | GB 2413078 A | 19-10-2005 |
| | | JP 5057783 B2 | 24-10-2012 |
| | | JP 2007517565 A | 05-07-2007 |
| | | PL 1694203 T3 | 29-01-2010 |
| | | PT 1694203 E | 18-11-2009 |
| | | SI 1694203 T1 | 29-01-2010 |
| | | US 2007270699 A1 | 22-11-2007 |
| | | US 2014039326 A1 | 06-02-2014 |
| | | WO 2005065533 A1 | 21-07-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **N. BAJWA et al.** Terahertz Imaging of Cutaneous Edema: Correlation With Magnetic Resonance Imaging in Burn Wounds. *IEEE Trans Biomed Eng,* 2017, vol. 64 (11), 2682-2694
- **G. G. HERNANDEZ-CARDOSO et al.** Terahertz imaging for early screening of diabetic foot syndrome: A proof of concept. *Sci Rep,* June 2017, vol. 7, 42124

- **Q. SUN ; Y. HE ; K. LIU ; S. FAN ; E. P. J. PARROTT ; E. PICKWELL-MACPHERSON.** Recent advances in terahertz technology for biomedical applications. *Quant Imaging Med Surg,* June 2017, vol. 7 (3), 345-355
- **S. I. ALEKSEEV ; I. SZABO ; M. C. ZISKIN.** Millimeter wave reflectivity used for measurement of skin hydration with different moisturizers. *Skin Research and Technology,* 2008, vol. 14 (4), 390-396